(19) Europäisches Patentamt / European Patent Office / Office européen des brevets

(11) **EP 3 653 185 A1**

(12) **EUROPEAN PATENT APPLICATION**
published in accordance with Art. 153(4) EPC

(43) Date of publication:
**20.05.2020  Bulletin 2020/21**

(21) Application number: **18845875.6**

(22) Date of filing: **28.06.2018**

(51) Int Cl.:
*A61F 13/42* (2006.01)      *A61F 13/49* (2006.01)
*G01N 27/22* (2006.01)      *G01N 29/44* (2006.01)

(86) International application number:
**PCT/KR2018/007317**

(87) International publication number:
**WO 2019/035552 (21.02.2019 Gazette 2019/08)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **14.08.2017  KR 20170103080**
**06.12.2017  KR 20170166571**

(71) Applicant: **Qui Inc.**
**Daejeon 34129 (KR)**

(72) Inventor: **KWAK, Byung Jae**
**Daejeon 34129 (KR)**

(74) Representative: **V.O.**
**P.O. Box 87930**
**2508 DH Den Haag (NL)**

(54) **EXCRETA DETECTION DEVICE FOR DIAPER AND EXCRETA DETECTION READER**

(57)    Provided are an excreta detection device that is for a diaper and capable of detecting excreta present in the diaper, and an excreta detection reader for transmitting and receiving signals to and from the excreta detection device for the diaper and notifying a user about whether excreta is present. Since the large scale of a resonant frequency is determined on the basis of a capacitor which is isolated from the excreta and not affected by the excreta and the small scale of the resonant frequency that changes due to the excreta may be determined on the basis of a capacitor having a permittivity that changes due to the excreta, the excreta may be detected even when a relatively narrow frequency band is swept. In addition, since excreta sensing is not performed at only a specific resonant frequency but is also performed by comparing resonant frequencies before and after discharge of excreta, the excreta sensing can be performed regardless of the state of the excreta.

Fig. 1

## Description

[TECHNICAL FIELD]

Embodiments of the inventive concept described herein relate to an excreta detection device for a diaper and an excreta detection reader and more particularly, relate to an excreta detection device for detecting a target material (e.g., excreta or the like) present in the diaper and an excreta detection reader for notifying a user of information about excreta.

[BACKGROUND ART]

Infants, old people, and patients who have limited mobility, who cannot treat discharge of excreta themselves, wear their diapers to solve discharge of excreta problems. Because most of users or the like, who wear their diapers, have deteriorated cognitive ability, deteriorated communication ability, or deteriorated physical ability, it is impossible for the users to replace the soiled diapers with new ones for themselves and the users are often incapable of notifying their caregivers or the like whether they have a discharge of excreta. When the users do not wear fresh diapers, diseases such as skin erythema and erosion can appear due to excreta which is present in the soiled diaper, and to prevent it, caregivers or the like should manually observe the outside of the diaper frequently using the sense of touch, sight, or smell to verify whether the users have a discharge of excreta. However, it is difficult for caregivers or the like to manually verify the states of the diapers frequently, and it is a very time consuming task for one caregiver or the like to verify discharge of excreta states of multiple users or the like frequently. In addition, as the sealing ability of diapers have been improving in recent years, it is more difficult to verify whether users have a discharge of excreta without opening the diapers the users are wearing. To address it, a device based on a semiconductor component for determining that excreta is present when a resonant frequency of a resonant circuit is changed as permittivity is changed by excreta and when a specific frequency is included in the changed resonant frequency range was invented. However, the device based on the semiconductor component has high unit cost of manufacture and requires power to operate. A device for measuring a change in resonant frequency based on a change in capacitance through a switching manner which is turned on/off by excreta may be used to detect excreta. However, because the device has a switch which is turned on/off to change capacitance depending on whether excreta is present, it has a complex structure and has high unit cost of manufacture. A device for detecting a change in resonant frequency based on a continuous change in permittivity of a capacitor by excreta may be used to detect excreta. However, because the difference in permittivity of the capacitor is very large depending on whether excreta is present, there is a need to sweep a frequency band of an unnecessarily wide bandwidth to determine the changed resonant frequency.

[DETAILED DESCRIPTION OF THE INVENTION]

[TECHINICAL PROBLEM]

Embodiments of the inventive concept may provide an excreta detection device for receiving wireless power without the necessity of having its own power supply to have a small size and have low unit cost of manufacture.

Embodiments of the inventive concept may provide an excreta detection device for determining a change in resonant frequency before and after discharge of excreta based on a change in capacitance of a capacitor due to excreta in the diaper to have a relatively simple structure without a switch structure.

Embodiments of the inventive concept may provide an excreta detection device which has a structure that allows a narrow frequency sweep range for resonant frequency detection, and an excreta detection reader usable together with the excreta detection device.

[TECHNICAL SOLUTION]

According to an exemplary embodiment, an excreta detection device may include at least one inductance element, at least one fixed capacitance element configured not to have a change in capacitance although the excreta is present in the diaper, and at least one variable capacitance element configured to be electrically connected with the at least one fixed capacitance element and have a change in capacitance when the excreta is present in the diaper. At least one first resonant frequency may be determined based on the at least one inductance element, the at least one fixed capacitance element, and the at least one variable capacitance element.

The at least one variable capacitance element may include an absorber that absorbs the excreta to change a capacitance of the at least one variable capacitance element and conductors separated by the absorber in between the conductors.

In addition, the at least one variable capacitance element may further include at least one insulating layer between the absorber and the at least one conductor to prevent the conductors from being directly exposed to the excreta absorbed by the absorber. In this case, the insulating layer may be present irrespective of whether the absorber is present or how the absorber is arranged.

The at least one variable capacitance element may have a change in capacitance, when the excreta is present in the diaper. At least one second resonant frequency may be determined based on the at least one inductance element, the at least one fixed capacitance element, and the at least one variable capacitance element, the capacitance of which is changed. The at least one first resonant frequency and the at least one second resonant frequency may be different from each other.

According to an exemplary embodiment, an excreta detection reader may include a power supply unit that generates a first alternating magnetic field , a frequency of which is changed to induce an electric current in the excreta detection device, a sensor unit that senses a parameter associated with a second alternating magnetic field generated by the induced electric current in the excreta detection device, a control unit that determines whether excreta is present, based on the parameter associated with the second alternating magnetic field, the parameter being sensed by the sensor unit, and an output unit that notifies a user whether the excreta is present.

The power supply unit may generate the first alternating magnetic field, the frequency of which is changed within a frequency band including at least one first resonant frequency and at least one second resonant frequency.

The parameter may be the amplitude of an induced current for each frequency, the amplitude of or an induced voltage for each frequency, or impedance for each frequency.

The control unit may determine a resonant frequency based on the value of the parameter and may compare the resonant frequency with at least one first resonant frequency or at least one second resonant frequency to determine whether the excreta is present.

The control unit may determine at least one resonant frequency before the excreta is present and at least one resonant frequency after the excreta is present, based on a value of the parameter, and may determine whether the excreta is present, based on the at least one resonant frequency before the excreta is present and the at least one resonant frequency after the excreta is present.

According to an exemplary embodiment, an apparatus for detecting a target material may include at least one fixed capacitance element configured not to have a change in capacitance although the target material is present and at least one variable capacitance element configured to be electrically connected with the at least one fixed capacitance element and have a change in capacitance when excreta is present in a diaper. At least one first resonant frequency may be determined based on the at least one fixed capacitance element and the at least one variable capacitance element.

[ADVANTAGEOUS EFFECTS OF THE INVENTION]

The excreta detection device according to the inventive concept may receive wireless power without the necessity of having its own power supply to have a small size and have low unit cost of manufacture.

The excreta detection device according to the inventive concept may determine a change in resonant frequency before and after discharge of excreta based on a change in capacitance of a capacitor (particularly a variable capacitor) due to excreta in the diaper to have a relatively simple structure without a switch structure.

The excreta detection device according to the inventive concept and the excreta detection reader usable together with the excreta detection device may have a structure capable of narrowing a frequency sweep range for measuring a resonant frequency.

[DESCRIPTION OF THE DRAWINGS]

FIG. 1 is a drawing illustrating an excreta detection device and an excreta detection reader according to an embodiment;
FIG. 2 is an example of a graph illustrating a parameter value sensed by a sensor unit of an excreta detection reader;
FIG. 3 is a drawing illustrating a conventional excreta detection device;
FIG. 4 is an example illustrating an excreta detection device according to an embodiment;
FIG. 5 is another example illustrating an excreta detection device according to an embodiment;
FIG. 6 is another example illustrating an excreta detection device according to an embodiment;
FIGS. 7 to 13 are drawings illustrating the structures of capacitance elements;
FIG. 14 illustrates an embodiment of using an interdigital capacitor shown in FIG. 13 as a variable capacitance element;
FIG. 15 illustrates an equivalent circuit for an embodiment shown in FIG. 14;
FIG. 16 illustrates an embodiment of using parasitic capacitance of an inductor as a variable capacitance element; and
FIG. 17 illustrates an equivalent circuit for an embodiment shown in FIG. 16.

[BEST MODE]

Hereinafter, a description will be given in detail of a specific embodiment among various embodiments of the inventive concept with reference to the accompanying drawings. However, this specific embodiment is restricted or limited to the inventive concept. Like reference numerals indicates like components without regard to denotations of the drawings, and a duplicated description will be omitted.

Embodiments of the inventive concept are applicable to technologies of detecting excreta present in a diaper and are not always limited to such technologies. In this case, those skilled in the art may easily derive technologies of detecting various materials from the technical scope of the inventive concept. For example, embodiments of the inventive concept are applicable to detecting whether any material having various forms, such as a liquid, a gas, and a solid, is present. Hereinafter, an embodiment of the inventive concept is exemplified as a target material is excreta present in the diaper.

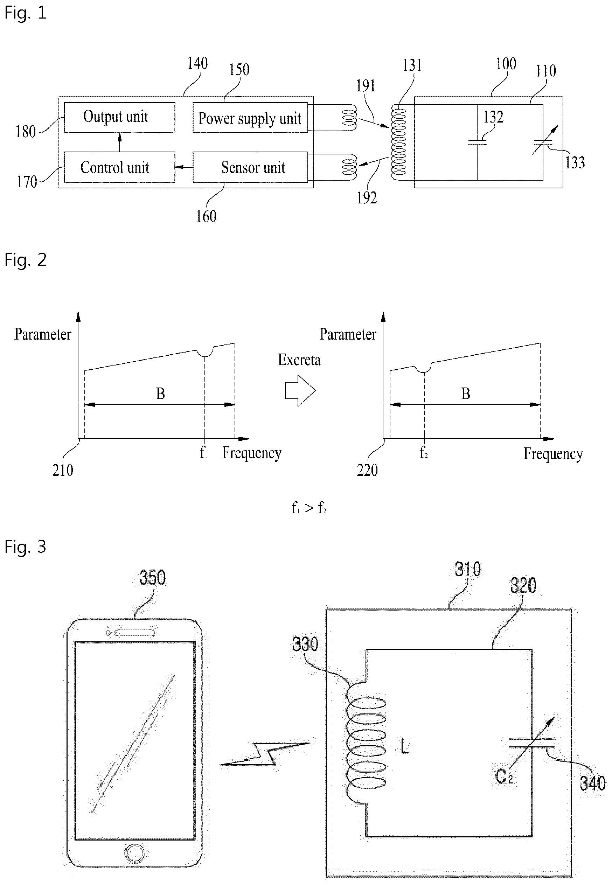

FIG. 1 is a drawing illustrating an excreta detection device and an excreta detection reader according to an embodiment.

Referring to FIG. 1, an excreta detection device 100 may include a resonant circuit 110. The resonant circuit 110 may include an inductance element 131, a fixed capacitance element 132, and a variable capacitance element 133.

When excreta is present in a diaper, the variable capacitance element 133 may have a change in capacitance. To this end, an absorber (not shown) which absorbs the excreta may be present between two conductors. That the absorber is present between the two conductors may mean that the absorber is located in a place where the absorber has an influence on the capacitance formed by the two conductors. In other words, that the absorber is present between the two conductors may include when the absorber is present around each conductor and is able to have an influence on the capacitance as well as when the absorber is present in a space between the conductors.

When the inductance element 131 is implemented in practice, a capacitance component (e.g., a parasitic capacitance element) is present in the inductance element 131. Because permeability which has an influence on an inductance of the inductance element 131 is a variable which is relatively less affected by excreta, although excreta is located near the inductance element, the inductance changes relatively less, but a capacitance of a capacitance component changes to the point of being unable to ignore it as permittivity is changed when the excreta is present near the capacitance component. To take advantage of the fact, instead of regarding the variable capacitance component that occurs in practical implementation of an inductance element 131 (e.g., a parasitic capacitance component) as an extra element in the design/arrangement, the variable capacitance component can be allowed to play a role as a variable capacitance element 133.

In this case, in a process of designing a specific element, an embodiment of the inventive concept may allow the element to have desired capacitance and inductance. For example, by intentionally structuring a conductor included in the specific element, an embodiment of the inventive concept may allow the element to have desired capacitance and inductance.

In the specification, the variable capacitance element 133 is defined as the term including a parasitic capacitance component in such a design.

Although the fixed capacitance element 132 is designed to be disposed on a location which is nearly unaffected by excreta and have a structure for minimizing an influence of excreta, or is waterproofed or disposed to be nearly unaffected by excreta, the capacitance of the fixed capacitance element may change minutely due to surrounding environments such as human fluid, humidity, and even excreta. Herein, in the specification, for convenience of description, it is described that a subtle change in capacitance of the fixed capacitance element is excluded and that the fixed capacitance element does not have a change in capacitance due to excreta.

The number of inductance elements and capacitance elements may be different from that shown in FIG. 1. Because there can be a variety of combinations of inductance elements and capacitance elements to obtain a desired resonant frequency, the number of inductance elements and capacitance elements may be varied according to a design.

The excreta detection reader 140 may include a power supply unit 150, a sensor unit 160, a control unit 170, and an output unit 180. It is shown that the excreta detection reader 140 shown in FIG. 1 includes the power supply unit 150, the sensor unit 160, the control unit 170, and the output unit 180, but at least one of the power supply unit 150, the sensor unit 160, the control unit 170, and the output unit 180 may be separated as a separate module. For example, the power supply unit 150 may be configured as a module separated from the sensor unit 160, the control unit 170, and the output unit 180.

The power supply unit 150 may allow an electric current to flow in a coil to generate a first alternating magnetic field 191 having a specific frequency band. The frequency band may be set to a band including a resonant frequency of the resonant circuit 110. The alternating magnetic field is a magnetic field produced by an alternating current (AC) that flows in a coil, and the magnitude and the direction change in time. A sinusoidal wave may flow in a coil to generate an alternating magnetic field.

The first alternating magnetic field 191 creates a time varying magnetic flux linkage that affects the inductance element 131 of the resonant circuit 110 and induces electric current in the resonant circuit 110. When the induced current flows in the resonant circuit 110, the inductance element 131 produces a second alternating magnetic field 192 different from the first alternating magnetic field 191.

Although the amplitude of the first alternating magnetic field 191 is constant in the frequency band, because the impedance of the resonant circuit 110 is varied for each frequency, the amplitude of the induced current is also varied for each frequency, and, as a result, the amplitude of the second alternating magnetic field 192 is not constant. Furthermore, the amplitude of the induced current is the highest at the resonant frequencies of the resonant circuit 110, and thus the second alternating magnetic field 192 generated by the induced current has the highest amplitude at the resonant frequencies of the resonant circuit. In other words, when the amplitude of the first alternating magnetic field 191 is constant in the frequency band, and when the frequency of the first alternating magnetic field 191 is swept in the frequency band and the second alternating magnetic field 191 is sensed, it may be verified that the second alternating magnetic field 192 has the highest amplitude at the resonant frequencies.

In this case, when a change in the amplitude of the second alternating magnetic field 192 is measured to determine the resonant frequency, the amplitude of the first alternating magnetic field 191, which is an input, may be kept constant. Furthermore, by making the quality factor (Q factor) of the resonant circuit 110 high, the characteristics that the amplitude of the second alternating magnetic field 192 changes near the resonant frequency may be clearly observed.

The coil of the sensor unit 160 may detect the second alternating magnetic field 192, and an electric current may be induced in the sensor unit 160 by the second alternating magnetic field 192. When the amplitude of the first alternating magnetic field 191 is constant, because it is possible to find the resonant frequency of the resonant circuit 110 using the amplitude of the second alternating magnetic field 192, the resonant frequency of the resonant circuit 110 may be determined when the current induced by the second alternating magnetic field 192 is analyzed. A description will be given of a detailed embodiment with reference to FIG. 2. Voltage, impedance, or the like as well as current may be used as a parameter for determining the resonant frequencies.

For the convenience of description, the coils of the power supply unit 150 and the sensor unit 160 are represented as being different from each other, but the power supply unit 150 and the sensor unit 160 may share the same coil.

In the specification, the 'first resonant frequency' may be a reference frequency indicating that there is no excreta and there may be one or more first resonant frequencies. When there is no excreta, the first resonant frequency may be a resonant frequency value determined theoretically or experimentally based on the inductance of an inductance element, the capacitance of a variable capacitance element, or the capacitance of a fixed capacitance element. In an example, the first resonant frequency may be determined by a manufacturer based on the values of the elements that comprise an excreta detection device or an excreta detection reader. In another example, a user may determine the first resonant frequency by directly entering a frequency value into the excreta detection reader or may determine the first resonant frequency through an action of pushing a 'no excreta' button and measuring a resonant frequency.

There may be one or more 'second resonant frequencies'. The second resonant frequency may be a reference frequency indicating that excreta is present. When excreta is present, the second resonant frequency may be a resonant frequency value determined theoretically or experimentally based on the inductance of an inductance element, the capacitance of a variable capacitance element, or the capacitance of a fixed capacitance element.

In an embodiment, when a resonant circuit, a capacitance of the variable capacitance element 133 of which is not changed because excreta is not present in a diaper, is called a first resonant circuit, a first resonant frequency of the first resonant circuit may be determined by the inductance element 131, the fixed capacitance element 132, and the variable capacitance element 133. In this case, when the power supply unit 150 generates the first alternating magnetic field 191, the resonant circuit 110 may generate the second alternating magnetic field 192 in response to it and the sensor unit 160 may sense the second alternating magnetic field 192. The control unit 170 may measure a resonant frequency at this time and may determine that the measured resonant frequency is the same as the first resonant frequency within a margin of error to determine that there is no excreta in the diaper.

When a resonant circuit, a capacitance of the variable capacitance element 133 of which is changed due to excreta present in the diaper, is call a second resonant circuit, because a second resonant frequency of the second resonant circuit is determined based on the inductance element 131, the fixed capacitance element 132, and the variable capacitance element 133, the capacitance of which is changed, the second resonant frequency may be different from the first resonant frequency.

The control unit 170 may measure the changed resonant frequency using information received from the sensor unit 160 and may determine that the measured resonant frequency is the same as the second resonant frequency within a margin of error to determine that there is excreta in the diaper. The control unit 170 may allow the output unit 180 to provide an excreta detection alarm to a user.

To decide whether the measured resonant frequency is identical to the first resonant frequency or to the second resonant frequency within a margin of error, the frequency band of the first alternating magnetic field 191 may be determined to include the first resonant frequency and the second resonant frequency.

Because the variable capacitance element 133 needs to change its capacitance when it is exposed to excreta, it may be located in a place where the excreta will be discharged. When a variable capacitance element portion except for an absorber and a resonant circuit portion except for the absorber come into direct contact with excreta, because the direct contact may lead to an unexpected change in capacitance or inductance or introduce a parasitic resistance component, the variable capacitance element portion except for the absorber and the resonant circuit portion except for the absorber may be insulated or waterproofed.

It is shown in FIG. 1 that conductors (metal plates) separated from each other are planar plates substantially parallel to each other, but an embodiment of the inventive concept may include any structure of separated metal plates capable of having capacitance. For the convenience of description, a description will be given of an inventive concept using planar plates parallel to each other. Herein, the "conductor" or "metal plate" is not necessarily limited to having a planar structure and refers to an object or circuit made of a conductive material having any structure. For example, it should be understood that a circuit manufactured with a conductive ink or a circuit formed of a conducive material through etching are included in the category of the 'conductor' or "metal plate" used in the specification.

FIG. 2 is an example of a graph illustrating a parameter value sensed by a sensor unit of an excreta detection reader.

In detail, a graph 210 of a parameter value of a first resonant circuit sensed by a sensor unit 160 and a graph 220 of a parameter value of a second resonant circuit sensed by the sensor unit 160 are shown.

In the graph 210 of the parameter value of the first resonant circuit sensed by the sensor unit 160, it may be observed that the rate of change in the parameter value at $f_1$ in a specific frequency band B is closest to 0. A control unit 170 may obtain the frequency where the rate of change in the parameter value is closest to 0. Because a point where the rate of change in the parameter value is closest to 0 represents a local minimum value in the graph shown in FIG. 2, the control unit 170 may determine a frequency at the point as a resonant frequency. As shown in FIG. 2, the control unit 170 may obtain $f_1$ when excreta is not present and may obtain $f_2$ when excreta is present.

In an example, when the measured resonant frequency $f_1$ and the resonant frequency $f_2$ measured later have a difference greater than a threshold, the control unit 170 may determine that excreta is present. For example, when a user measures a resonant frequency of an excreta detection device using an excreta detection reader to obtain 1 MHz and then measures a resonant frequency of the excreta detection device to obtain 2 MHz, because the difference between the measurements are greater than or equal to 0.5 MHz which is a predetermined threshold, the control unit 170 may determine that excreta is present.

The user may make sure that excreta is not present in a diaper and measure the resonant frequency so that the control unit 170 obtains $f_1$. For example, the user may enter 'no excreta' via an input unit (not shown) of the excreta detection reader and may measure a resonant frequency, so that $f_1$ becomes a resonant frequency when excreta is not present. Thereafter, when the user measures a resonant frequency when excreta is present in the diaper using the excreta detection reader, the control unit 170 may obtain $f_2$ and may compare $f_2$ with $f_1$ to determine whether excreta is present.

In an example, the control unit 170 may determine that $f_1$ which is the measured resonant frequency is the same as a first resonant frequency of the designed first resonant circuit within a predetermined margin of error to determine that excreta is not present in the diaper. For example, when the first resonant frequency is 1 MHz

and when $f_1$ measured by the excreta detection reader is 1.02 MHz, the control unit 170 may determine that the first resonant frequency and $f_1$ are the same as each other within a predetermined margin of error 2% to determine that excreta is not present in the diaper. Likewise, the control unit 170 may determine that $f_2$ which is the measured resonant frequency is the same as the expected second resonant frequency when variable capacitance is changed by excreta within a predetermined margin of error to determine that excreta is present in the diaper. For example, when the expected second resonant frequency is 3 MHz and when $f_2$ measured by the excreta detection reader is 3.03 MHz, the control unit 170 may determine that the second resonant frequency and $f_2$ are the same as each other within a predetermined margin of error 1% to determine that excreta is present in the diaper.

In an embodiment, the control unit 170 may determine a resonant frequency measured after the user pushes, for example, a 'no excreta' button in a desired time as the first resonant frequency, other than determining a resonant frequency theoretically calculated based on a theoretical element value or a real element value of a variable capacitance element, a fixed capacitance element, or an inductance element as the first resonant frequency or the second resonant frequency.

In an example, the control unit 170 may determine the frequency where a rate of change in parameter value is closest to 0 and may compare the frequency with a frequency threshold preset to determine whether excreta is present. For example, the control unit 170 may determine that $f_1$ measured as 1 MHz is greater than a predetermined frequency threshold 0.9 MHz to determine that excreta is not present.

In an example, the control unit 170 may determine whether $f_1$ is within a predetermined frequency threshold range to determine that excreta is not present. For example, the control unit 170 may determine that $f_1$ measured as 1 MHz is greater than 0.9 MHz and is less than 1.2 MHz, which is a predetermined frequency range, to determine that excreta is not present.

An output unit 180 may provide information indicating that excreta is not present to the user using a display, a sound, or the like.

It is shown in FIG. 2 that $f_1 > f_2$, but it is possible that $f_1 < f_2$ depending on a design of a resonant circuit. An embodiment of measuring a resonant frequency using a parameter value and determining whether excreta is present, for example, a method for determining a frequency where there is the highest rate of change in parameter value as a resonant frequency, a method for determining a frequency that generates a smallest parameter value as a resonant frequency, or a method for determining a frequency that generates a highest parameter value as a resonant frequency, other than a method for determining a frequency at which the rate of change in parameter value is closest to 0 in a method for finding the resonant frequency, may correspond to an embodiment of the inventive concept.

In an embodiment, the excreta detection reader may measure several resonant frequencies in each of states before and after discharge of excreta using the fact that one circuit may have several resonant frequencies depending on the structure of the resonant circuit and may compare the difference between the several resonant frequencies with a predetermined threshold to determine whether the diaper is in before discharge of excreta state or after discharge of excreta state.

As an example, when the first resonant circuit generates two resonant frequencies, the excreta detection reader may measure the two resonant frequencies of the first resonant circuit and may compare the difference between the two resonant frequencies with a predetermined threshold to determine that excreta is not present. In the same method, the excreta detection reader may compare the difference between at least two resonant frequencies of the second resonant circuit with a predetermined threshold to determine that excreta is present in the diaper.

When a physical external force is applied to a resonant circuit, an inductance element, a capacitance element, or the like, which has an influence on each of two resonant frequencies in common may be physically deformed. In this case, each of the several resonant frequencies in the first resonant circuit or the second resonant circuit may have a large change in value by the deformation of the inductance element, the capacitance element, or the like, whereas the difference between each of the several resonant frequencies may have a small change in value making it possible to detect excreta more reliably.

Furthermore, embodiments of the inventive concept may be modified in more various manners. For example, as in the case that there are two resonant frequencies before excreta is present but there is one resonant frequency when excreta is present, the number of resonant frequencies may change. An embodiment of the inventive concept may detect a change in the number of resonant frequencies. On the other hand, the case where there is one resonant frequency before excreta is present but there are two resonant frequencies when excreta is present is an embodiment of the inventive concept.

In addition, frequency shift of a resonant frequency as well as a change in the number of resonant frequencies may be detected. For example, two resonant frequencies before excreta is present may be decreased or increased while the interval between the two resonant frequencies is maintained when excreta is present, and an embodiment of the inventive concept is applicable to such a case.

FIG. 3 is a drawing illustrating a conventional excreta detection device.

Referring to FIG. 3, a conventional excreta detection device 310 may include a resonant circuit 320. The resonant circuit 320 may include an inductor 330 and a variable capacitor 340. The variable capacitor 340 may include an absorber, permittivity of which is changed when exposed to excreta. The variable capacitor 340 may have a change in capacitance by a change in permittivity. When the capacitance of the variable capacitor 340 changes, a resonant frequency determined by the inductor 330 and the variable capacitor 340 may change. An excreta detection reader 350 may measure the changed resonant frequency of the resonant circuit 320 and may determine whether excreta is present in a diaper. For example, when permittivity in the variable capacitor 340 increases by a factor of 100 due to excreta, the capacitance of the variable capacitor 340 increases by a factor of 100 according to Equation 1 below.

$$[\text{Equation 1}]$$

$$C_v = \varepsilon S/d$$

$\varepsilon$ denotes permittivity of the material between the metal plates, d denotes the distance between the metal plates, and S denotes the area of the metal plate. In this case, because the resonant frequency is represented as Equation 2 below, when capacitance increases by a factor of 100, the resonant frequency decreases by a factor of 1/10 due to excreta.

$$[\text{Equation 2}]$$

$$f_r = 1/\{2\pi(LC_v)^{1/2}\}$$

For example, when an inductance of the inductor 330 is 1 mH and when a capacitance of the variable capacitor 340 before excreta is present is 1 $\mu$F, the resonant frequency is 5033 Hz by Equation 2 above. When the capacitance of the variable capacitor 340 increases by a factor of 100 because excreta is present in the diaper, the resonant frequency is 503.3 Hz by Equation 2 above.

In general, when a dielectric which has an influence on capacitance is affected by excreta and changes in permittivity, because it is possible that the permittivity can increase dozens of times, the resonant frequency may greatly decrease. The excreta detection reader 350 may measure both of a first resonant frequency when excreta is not present in the diaper and a second resonant frequency when excreta is present in the diaper. Thus, the excreta detection reader 350 should perform a frequency sweep in a frequency range that includes the first resonant frequency and the second resonant frequency. In this case, when the resonant frequency greatly decreases as the permittivity increases dozens of times, the frequency range to be swept may become excessively wide and it will not only make the implementation of the excreta detection reader 350 complicated but also make the excreta detection speed slow.

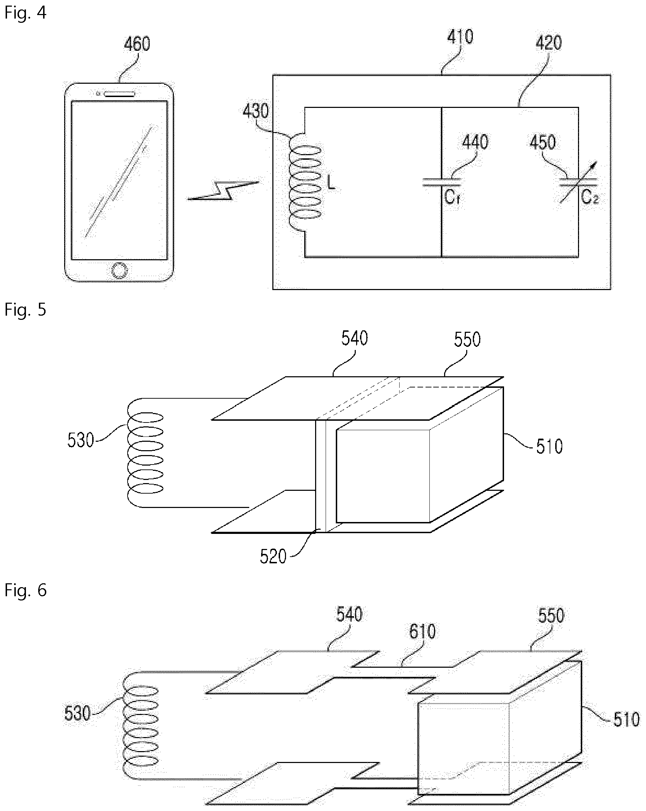

FIG. 4 is an example illustrating an excreta detection device according to an embodiment.

Referring to FIG. 4, an excreta detection device 410 according to an embodiment of the inventive concept may include a resonant circuit 420. The resonant circuit 420 may include an inductance element 430, a fixed capacitance element 440, and a variable capacitance element 450. For the convenience of description, one inductance element 430, one fixed capacitance element 440, and one variable capacitance element 450 are shown in FIG. 4, but the resonant circuit 420 may include one or more elements of each type. The resonant circuit may be designed through a parallel connection, a serial connection, or a combination thereof using one or more elements of each type, but a description will be given of an embodiment of the inventive concept using a resonant circuit 410 where the elements are connected in parallel for convenience of description.

The resonant frequency of a resonant circuit 420 may be determined by the inductance element 430, the fixed capacitance element 440, and the variable capacitance element 450 according to Equation 3 below.

[Equation 3]

$$f_r = 1/\{2\pi(LC_f+C_v)^{1/2}\}$$

When the capacitance of the variable capacitance element 450 is significantly less than the capacitance of the fixed capacitance element 440, a first resonant frequency may be represented as Equation 4 below.

[Equation 4]

$$f_r \doteqdot 1/\{2\pi(LC_f)^{1/2}\} \quad (C_f \gg C_v)$$

For example, when the inductance is 1 mH, when the capacitance of the fixed capacitance element 440 is 1 μF, and when the capacitance of the variable capacitance element 450 is 1 nF, the first resonance frequency before excreta is present will be about 5030 Hz. In other words, the first resonant frequency may be determined by the fixed capacitance element 450. To make the capacitance of the variable capacitance element 450 significantly less than the capacitance of the fixed capacitance element 440, the area of the parallel metal plates of the variable capacitance element 450 may be made significantly smaller than that of the parallel metal plates of the fixed capacitance element 440 or the distance between the parallel metal plates of the variable capacitance element 450 may be made significantly longer than that between the parallel metal plates of the fixed capacitance element 440 (in this case, that the area of the metal plates are made smaller or that the distance between the metal plates are made longer may mean that some design decisions are made to create an effect in which the area of the metal plates are smaller or the distance between the metal plates are longer in an equivalent circuit model, and the detailed method of achieving the effect may vary depending on the method of actually implementing the variable capacitor).

Although excreta is present in a diaper, the fixed capacitance element 440 has no change in capacitance. Thus, when excreta is present in the diaper, because only the capacitance of the variable capacitance element 450 is changed, compared with a conventional excreta detection device, the change of the resonant frequency by excreta may be reduced.

For example, when permittivity of the dielectric material around the variable capacitance element 450 increases by a factor of 100 because excreta is present in the diaper, because the capacitance of the variable capacitance element 450 increases by a factor of 100 to be 100 nF, the resonant frequency when excreta is present in the diaper may be about 4799 Hz by Equation 1 above. When the capacitance of the variable capacitance element 450 increases by a factor of 100 due to excreta, the resonant frequency of the conventional excreta detection device may decrease by 90%, whereas the resonant frequency of the excreta detection device 410 according to an embodiment of the inventive concept may decrease by 4.59% thanks to the fixed capacitance element 440. Thus, discharge of excreta because it is possible to measure the resonant frequencies before and after discharge of excreta with a small frequency sweep range, the excreta detection speed may be enhanced.

As another example, when the variable capacitance element 450 is designed to include an absorber and when permittivity of the absorber of the variable capacitance element 450 increases by a factor of 100 because excreta is present in the diaper, because the capacitance of the variable capacitance element 450 increases by a factor of 100 to be 100 nF, the resonant frequency when excreta is present in the diaper may be about 4799 Hz by Equation 1. When the permittivity of the variable capacitance element 450 increases by a factor of 100 due to excreta, the resonant frequency of the conventional excreta detection device decreases by 90%, whereas the resonant frequency of the excreta detection device 410 according to an embodiment of the inventive concept may decrease by 4.59% thanks to the fixed capacitance element 440. Thus, discharge of excreta because it is possible to measure the resonant frequencies before and after discharge of excreta with a small frequency sweep range, the excreta detection speed may be enhanced.

As described above, by making the magnitude of the capacitance of the variable capacitance element 450 significantly less than that of the capacitance of the fixed capacitance element 440, the fixed capacitance element 440 may be used to determine the first resonant frequency and the variable capacitance element 450 may be used to determine the second resonant frequency.

The excreta detection reader 460 may be a separate device for detecting excreta but may also be a mobile device, such as a smartphone, a PDA, or a tablet PC, having the above-mentioned functions.

FIG. 5 is another example illustrating an excreta detection device according to an embodiment.

Referring to FIG. 5, a variable capacitance element 550 may further include an absorber 510 for absorbing excreta. The absorber 510 may be located between the parallel metal plates of the variable capacitance element 550.

The 'separated metal plates' in the inventive concept may include metal plates which are substantially parallel to each other and may also include any form of metal plates capable of having capacitance such as coaxial cylindrical metal plates parallel to each other, metal plates that are separated from each other and are arranged in the form of rolls, and metal plates of any shape that are parallel to each other, as well as the plane-shaped metal plates parallel to each other.

That there is an absorber between two metal plates may mean that the absorber is located in a place where the absorber may have an influence on the capacitance formed by the two metal plates. In other words, that there is an absorber between two metal plates may mean that the absorber located in the space between the metal plates, but it may also mean that the absorber is present around the metal plates and is able to have an influence on the capacitance.

The absorber 510 may be extended to the outside of parallel metal plates to make it absorb the excreta in the diaper more efficiently. For example, even when the excreta is not located in the vicinity of the variable capacitance element 550, the excreta absorbed by the absorber portion extended to the outside of the parallel metal plates may soak through the absorber and reach the absorber portion between the parallel metal plates and change its permittivity.

Because the variable capacitance element 550 is is supposed to be affected by excreta, it may be exposed to excreta without an insulator.

In an example, when excreta comes into a direct contact with the variable capacitance element 550, because the conductive property of the excreta may lead to an unexpected change in parasitic capacitance or inductance, or introduce a parasitic resistance component, the parallel metal plate may be insulated or waterproofed to prevent it.

In an example, at least a part of the resonant circuit except for the absorber 510 may be insulated or waterproofed to prevent the at least a part of the resonant circuit except for the absorber 510 from being affected by excreta. As shown in FIG. 5, the fixed capacitance element 540 may be in the form of being integrally connected with the variable capacitance element 550. In this case, when the absorber 510 between the variable capacitance elements 550 absorbs excreta, because permittivity of the fixed capacitance element 540 should not be changed, there may be a wall separating the fixed capacitance element 540 and the variable capacitance element 550. In other words, reliability of the permittivity of the fixed capacitance element 540 may be maintained through the separating wall 520.

If the volume of the absorber 510 expands by absorbing excreta, the distance between the metal plates parallel to each other is increased, and the capacitance of the variable capacitance element 550 may be decreased according to Equation 1. The distance between the metal plates may be increased by the expansion of the absorber 510, and the amount of increase in the distance between the metal plates may depend on the expansion rate of the absorber, the excreta absorption rate of the absorber, or the resistance of the two metal plates parallel to each other to the expansion of the absorber 510. For example, even when the distance between the metal plates is increased by a factor of 2, if the permittivity increases dozens of times, the capacitance increases.

There is actually a dielectric between the parallel metal plates of the fixed capacitance element 540, but the dielectric of the fixed capacitance element 540 is omitted in FIG. 5 to emphasize that the portion where permittivity is changed is only in the variable capacitance element 550 in the inventive concept.

FIG. 6 is another example illustrating an excreta detection device according to an embodiment.

Referring to FIG. 6, a fixed capacitance element 540 and a variable capacitance element 550 may be electrically connected to each other by a connection portion 610. When the fixed capacitance element 540 and the variable capacitance element 550 are located within a short distance, contrary to the intention that only the variable capacitance element 550 is allowed to change its permittivity as the absorber 510 absorbs excreta, the fixed capacitance element 540 within a short distance may also be affected by the excreta and its permittivity may be changed. To prevent this from happening, the inductance element 530 and the fixed capacitance element 540 may be located away from the variable capacitance element 530 and from the location where excreta is discharged so that the inductance element 530 and the fixed capacitance element 540 are not affected by the excreta. In an example, at least a part of the resonant circuit except for the absorber 510 may be waterproofed to prevent the at least a part of the resonant circuit portion except for the absorber 510 from being affected by excreta.

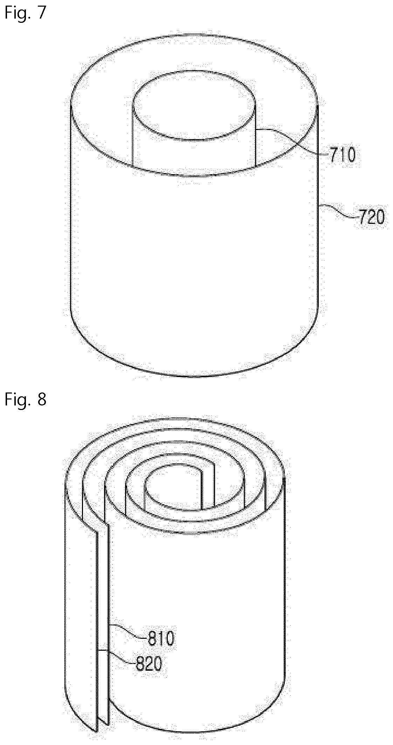

FIGS. 7 to 13 are drawings illustrating the structures of capacitance elements.

FIG. 7 illustrates the structure of a capacitance element where cylindrical plates are arranged in parallel to each other. Referring to FIG. 7, two cylindrical plates 710 and 720, each of which has a different diameter, may be arranged in parallel to each other.

FIG. 8 illustrates the structure of a capacitance element in the form of a roll. Referring to FIG. 8, at least two plates 810 and 820 may have a form where they are wound alternatively.

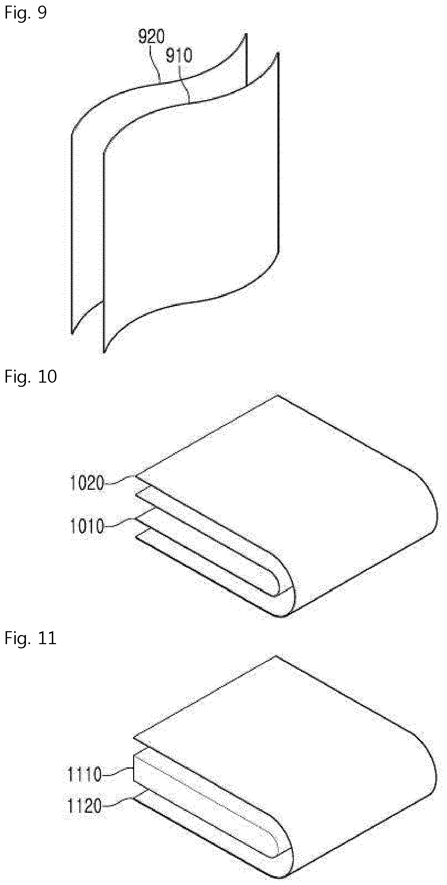

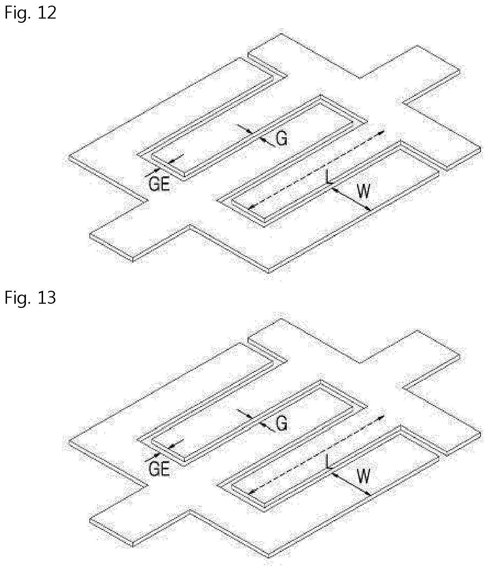

FIG. 9 illustrates the structure of a capacitance element where metal plates 910 and 920, each of which having an arbitrary curved surface shape, are arranged in parallel to each other. FIG. 10 is a drawing illustrating the structure of a capacitance element that consists of two bent parallel plates 1010 and 1020. FIG. 11 is a drawing illustrating the structure of a capacitance element that consists of a thick conductive plate 1110 and a bent conductive plate 1120. FIG. 12 is a drawing illustrating the structure of a capacitance element that consists of a thin conductive plate 1210 and a bent conductive plate 1220. FIG. 13 is a drawing illustrating the structure of a printable capacitance element that consists of conductive plates of a concave and convex shape.

FIG. 13 is a drawing illustrating a capacitance element of a concave and convex shape (a finger shape). The capacitance may be controlled by using gap G, end gap ($G_E$), width W, length L, and the number of fingers as variables. The capacitance element of a concave and convex shape shown in FIG. 13 is called an interdigital capacitor.

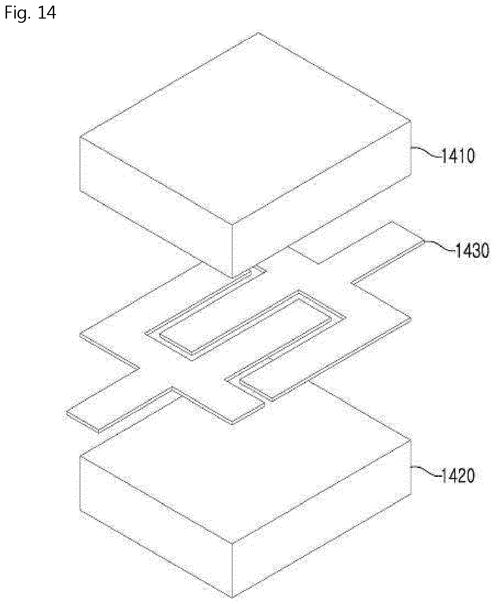

FIG. 14 illustrates an embodiment of using an interdigital capacitor shown in FIG. 13 as a variable capacitance element.

Referring to FIG. 14, an excreta detector of the inventive concept does not include a separate absorber. When excreta is absorbed by absorbers 1410 and 1420 of a diaper, there may occur a change in permittivity of the absorbers 1410 and 1420 of the diaper. The change in permittivity of the absorbers 1410 and 1420 of the diaper results in a change of the capacitance of the interdigital capacitor 1430 used as a variable capacitor. The excreta detector of the inventive concept may detect the changed capacitance of the interdigital capacitor 1430.

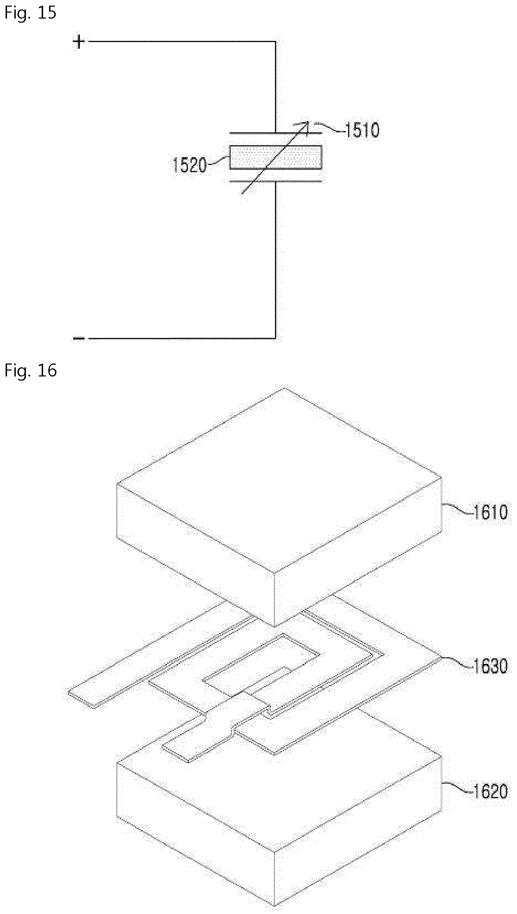

FIG. 15 illustrates an equivalent circuit of the embodiment shown in FIG. 14. Referring to FIG. 15, the interdigital capacitor 1430 may be represented equivalently as a variable capacitor 1510 in FIG. 15. Absorbers 1410 and 1420 of a diaper may be represented as a dielectric 1520 of the variable capacitor 1510 in the equivalent circuit. Thus, a change in permittivity of the absorbers 1410 and 1420 of a diaper may be represented by a change in permittivity of the dielectric 1520 of the variable capacitor 1510 of the equivalent circuit.

FIG. 16 illustrates an embodiment of using parasitic capacitance of an inductor as a variable capacitance element.

Referring to FIG. 16, an excreta detector of the inventive concept does not include a separate absorber. When excreta is absorbed by absorbers 1610 and 1620 of a diaper, there may occur a change in permittivity of the absorbers 1610 and 1620 of the diaper. The change in permittivity of the absorbers 1610 and 1620 of the diaper results in a change of the parasitic capacitance of the inductor 1630 used as a variable capacitor. The excreta detector of the inventive concept may detect the changed capacitance of the parasitic capacitance of the inductor 1630.

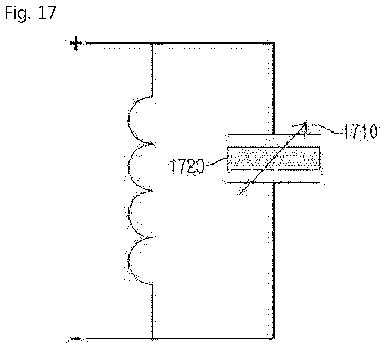

FIG. 17 illustrates an equivalent circuit of the embodiment shown in FIG. 16. Referring to FIG. 17, the parasitic capacitance of inductor 1630 may be represented equivalently as a variable capacitor 1710 in FIG. 17. Absorbers 1610 and 1620 of a diaper may be represented as a dielectric 1720 of the variable capacitor 1710 in the equivalent circuit. Thus, a change in permittivity of the absorbers 1610 and 1620 of a diaper may be represented by a change in permittivity of the dielectric 1720 of the variable capacitor 1710.

[MODE FOR INVENTION]

Although the inventive concept is given in detail of the limited embodiment, the inventive concept is not limited to the above-mentioned embodiment. Those skilled in the art will appreciate that various modifications, additions and substitutions are possible without departing from the scope and spirit of the inventive concept as disclosed in the accompanying claims.

**Claims**

1. An apparatus for detecting excreta from a diaper, the apparatus comprising:

   at least one inductance element;
   at least one fixed capacitance element configured not to have a change in capacitance although the excreta is present in the diaper; and
   at least one variable capacitance element configured to be electrically connected with the at least one fixed capacitance element and have a change in capacitance when the excreta is present in the diaper,
   wherein at least one first resonant frequency is determined based on the at least one inductance element, the at least one fixed capacitance element, and the at least one variable capacitance element.

2. The apparatus of claim 1, wherein the at least one variable capacitance element includes:

   an absorber configured to absorb the excreta to change a capacitance of the at least one variable capacitance element; and
   at least one conductor separated by the absorber in between the at least one conductor.

3. The apparatus of claim 2, wherein the at least one variable capacitance element further includes:
   at least one insulating layer between the absorber and the at least one conductor to prevent the at least one conductor from being directly exposed to the excreta absorbed by the absorber.

4. The apparatus of claim 1, wherein the at least one variable capacitance element has a change in capacitance, when the excreta is present in the diaper, wherein at least one second resonant frequency is determined based on the at least one inductance element, the at least one fixed capacitance element, and the at least one variable capacitance element, the capacitance of which is changed, and wherein the at least one first resonant frequency and the at least one second resonant frequency are different from each other.

5. An excreta detection reader, comprising:

a power supply unit configured to generate a first alternating magnetic field, a frequency of which is changed to induce an electric current in an excreta detection device according to any one of claims 1-4;

a sensor unit configured to sense a parameter associated with a second alternating magnetic field generated by the induced electric current in the excreta detection device;

a control unit configured to determine whether excreta is present, based on the parameter associated with the second alternating magnetic field, the parameter being sensed by the sensor unit; and

an output unit configured to notify a user whether the excreta is present.

6. The excreta detection reader of claim 5, wherein the power supply unit generates the first alternating magnetic field, the frequency of which is changed within a frequency band including at least one first resonant frequency and at least one second resonant frequency.

7. The excreta detection reader of claim 5, wherein the parameter is the amplitude of an induced current for each frequency, the amplitude of an induced voltage for each frequency, or impedance for each frequency.

8. The excreta detection reader of claim 5, wherein the control unit determines a resonant frequency based on a value of the parameter and compares the resonant frequency with at least one first resonant frequency or at least one second resonant frequency to determine whether the excreta is present.

9. The excreta detection reader of claim 5, wherein the control unit determines at least one resonant frequency before the excreta is present and at least one resonant frequency after the excreta is present, based on a value of the parameter, and determines whether the excreta is present, based on the at least one resonant frequency before the excreta is present and the at least one resonant frequency after the excreta is present.

10. An apparatus for detecting presence of a target material, the apparatus comprising:

at least one fixed capacitance element configured not to have a change in capacitance although the target material is present; and

at least one variable capacitance element configured to be electrically connected with the at least one fixed capacitance element and have a change in capacitance when the target material is present,

wherein at least one first resonant frequency is determined based on the at least one fixed capacitance element and the at least one variable capacitance element.

Fig. 1

Fig. 2

$f_1 > f_2$

Fig. 3

Fig. 4

Fig. 5

Fig. 6

Fig. 7

710

720

Fig. 8

810
820

Fig. 9

920 910

Fig. 10

1020
1010

Fig. 11

1110
1120

Fig. 12

Fig. 13

Fig. 14

1410

1430

1420

Fig. 15

Fig. 16

Fig. 17

## INTERNATIONAL SEARCH REPORT

| International application No. |
| --- |
| PCT/KR2018/007317 |

| A. CLASSIFICATION OF SUBJECT MATTER |
| --- |
| *A61F 13/42(2006.01)i, A61F 13/49(2006.01)i, G01N 27/22(2006.01)i, G01N 29/44(2006.01)i* |
| According to International Patent Classification (IPC) or to both national classification and IPC |

| B. FIELDS SEARCHED |
| --- |
| Minimum documentation searched (classification system followed by classification symbols) |
| A61F 13/42; A61F 13/15; G01N 25/26; G01R 27/26; G01N 21/3554; G01N 27/22; H04Q 5/22; G01N 21/3577; A61F 13/49; G01N 29/44 |
| Documentation searched other than minimum documentation to the extent that such documents are included in the fields searched<br>Korean Utility models and applications for Utility models: IPC as above<br>Japanese Utility models and applications for Utility models: IPC as above |
| Electronic data base consulted during the international search (name of data base and, where practicable, search terms used)<br>eKOMPASS (KIPO internal) & Keywords: diaper, manure detection, inductance element, fixed capacitance element, variable capacitance element, first resonance frequency, reader, power source part, sensor part, first alternating magnetic field, second alternating magnetic field, control part, output part |

| C. DOCUMENTS CONSIDERED TO BE RELEVANT | | |
| --- | --- | --- |
| Category* | Citation of document, with indication, where appropriate, of the relevant passages | Relevant to claim No. |
| X | US 2014-0296808 A1 (CURRAN, P. et al.) 02 October 2014<br>See abstract; claims 1-53; paragraphs [0048], [0051], [0058], [0059], [0063], [0076]-[0087], [0095], [0118]; and figures 1a-11a. | 1-10 |
| A | US 8978452 B2 (JOHNSON, J. M. et al.) 17 March 2015<br>See the entire document. | 1-10 |
| A | US 2004-0036484 A1 (TAMAI, S.) 26 February 2004<br>See the entire document. | 1-10 |
| A | KR 10-2012-0112500 A (KIMBERLY-CLARK WORLDWIDE, INC.) 11 October 2012<br>See the entire document. | 1-10 |
| A | US 7551058 B1 (JOHNSON, E. A. et al.) 23 June 2009<br>See the entire document. | 1-10 |

| ☐ Further documents are listed in the continuation of Box C. | ☒ See patent family annex. |
| --- | --- |

| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |
| --- | --- |
| Date of the actual completion of the international search<br><br>21 SEPTEMBER 2018 (21.09.2018) | Date of mailing of the international search report<br><br>**21 SEPTEMBER 2018 (21.09.2018)** |
| Name and mailing address of the ISA/KR<br>Korean Intellectual Property Office<br>Government Complex Daejeon Building 4, 189, Cheongsa-ro, Seo-gu, Daejeon, 35208, Republic of Korea<br>Facsimile No. +82-42-481-8578 | Authorized officer<br><br><br>Telephone No. |

Form PCT/ISA/210 (second sheet) (January 2015)

INTERNATIONAL SEARCH REPORT
Information on patent family members

International application No.

**PCT/KR2018/007317**

| Patent document cited in search report | Publication date | Patent family member | Publication date |
|---|---|---|---|
| US 2014-0296808 A1 | 02/10/2014 | AU 2012-289823 A1 | 02/05/2013 |
| | | AU 2012-289823 B2 | 28/05/2015 |
| | | CA 2876622 A1 | 07/02/2013 |
| | | CA 2876622 C | 05/06/2018 |
| | | EP 2739254 A1 | 11/06/2014 |
| | | EP 2739254 B1 | 16/11/2016 |
| | | US 9314381 B2 | 19/04/2016 |
| | | WO 2013-016765 A1 | 07/02/2013 |
| US 8978452 B2 | 17/03/2015 | BR 112014003174 A2 | 21/02/2017 |
| | | CA 2844826 A1 | 14/02/2013 |
| | | CN 103797360 A | 14/05/2014 |
| | | CN 103797360 B | 25/11/2015 |
| | | EP 2742343 A2 | 18/06/2014 |
| | | JP 2014-529732 A | 13/11/2014 |
| | | JP 2017-207503 A | 24/11/2017 |
| | | JP 6263472 B2 | 17/01/2018 |
| | | TW 201312104 A | 16/03/2013 |
| | | TW I612299 B | 21/01/2018 |
| | | US 2013-0036802 A1 | 14/02/2013 |
| | | US 2015-0148762 A1 | 28/05/2015 |
| | | US 9782302 B2 | 10/10/2017 |
| | | WO 2013-023054 A2 | 14/02/2013 |
| | | WO 2013-023054 A3 | 10/05/2013 |
| US 2004-0036484 A1 | 26/02/2004 | JP 2004-085277 A | 18/03/2004 |
| | | JP 3717068 B2 | 16/11/2005 |
| | | US 7049969 B2 | 23/05/2006 |
| KR 10-2012-0112500 A | 11/10/2012 | AR 079176 A1 | 28/12/2011 |
| | | AR 079480 A1 | 25/01/2012 |
| | | AU 2009-334407 A1 | 08/07/2010 |
| | | AU 2009-334407 B2 | 22/10/2015 |
| | | AU 2010-332429 A1 | 31/05/2012 |
| | | AU 2010-332429 B2 | 04/02/2016 |
| | | AU 2010-337961 A1 | 14/06/2012 |
| | | AU 2010-337961 B2 | 26/05/2016 |
| | | BR 112012013127 A2 | 21/03/2017 |
| | | BR 112012015599 A2 | 22/03/2016 |
| | | CN 102271641 A | 07/12/2011 |
| | | CN 102271641 B | 05/11/2014 |
| | | CN 102630156 A | 08/08/2012 |
| | | CN 102630156 B | 02/12/2015 |
| | | CN 102695488 A | 26/09/2012 |
| | | CN 102695488 B | 22/06/2016 |
| | | EP 2376047 A2 | 19/10/2011 |
| | | EP 2376047 A4 | 25/04/2012 |
| | | EP 2376047 B1 | 18/03/2015 |
| | | EP 2512392 A2 | 24/10/2012 |

Form PCT/ISA/210 (patent family annex) (January 2015)

INTERNATIONAL SEARCH REPORT
Information on patent family members

International application No.

**PCT/KR2018/007317**

| Patent document cited in search report | Publication date | Patent family member | Publication date |
|---|---|---|---|
| | | EP 2512392 A4 | 10/07/2013 |
| | | EP 2519207 A2 | 07/11/2012 |
| | | EP 2519207 A4 | 10/07/2013 |
| | | EP 2519207 B1 | 18/04/2018 |
| | | KR 10-1749354 B1 | 20/06/2017 |
| | | KR 10-1753437 B1 | 03/07/2017 |
| | | KR 10-1768242 B1 | 14/08/2017 |
| | | KR 10-2011-0107330 A | 30/09/2011 |
| | | KR 10-2012-0104240 A | 20/09/2012 |
| | | MX 2011007017 A | 06/09/2011 |
| | | MX 2012006848 A | 17/07/2012 |
| | | MX 2012007629 A | 06/11/2012 |
| | | RU 2011131921 A | 10/02/2013 |
| | | RU 2012129670 A | 27/01/2014 |
| | | RU 2012132222 A | 10/02/2014 |
| | | RU 2506939 C2 | 20/02/2014 |
| | | RU 2553008 C2 | 10/06/2015 |
| | | RU 2571808 C2 | 20/12/2015 |
| | | US 2010-0164733 A1 | 01/07/2010 |
| | | US 2010-0168694 A1 | 01/07/2010 |
| | | US 2010-0168702 A1 | 01/07/2010 |
| | | US 8274393 B2 | 25/09/2012 |
| | | US 8866624 B2 | 21/10/2014 |
| | | WO 2010-076679 A2 | 08/07/2010 |
| | | WO 2010-076679 A3 | 28/10/2010 |
| | | WO 2011-073825 A2 | 23/06/2011 |
| | | WO 2011-073825 A3 | 17/11/2011 |
| | | WO 2011-080607 A2 | 07/07/2011 |
| | | WO 2011-080607 A3 | 17/11/2011 |
| US 7551058 B1 | 23/06/2009 | US 7038470 B1 | 02/05/2006 |

Form PCT/ISA/210 (patent family annex) (January 2015)